# EUROPEAN PATENT SPECIFICATION

(11) **EP 3 603 572 B1**
(45) Date of publication and mention of the grant of the patent: **19.01.2022**
(21) Application number: 18774502.1
(22) Date of filing: 30.03.2018
(51) Int. Cl.: A61F 2/06, A61L 27/04, A61L 31/08, A61L 33/02, A61L 27/06, A61L 27/18, A61L 27/30, A61L 27/50, A61L 31/06, A61L 33/00, A61L 33/06

(54) **METHOD FOR ENDOTHELIALIZING VASCULAR PROSTHESES**
VERFAHREN ZUR ENDOTHELIALISIERUNG VON GEFÄSSPROTHESEN
PROCÉDÉ D'ENDOTHÉLISATION DE PROTHÈSES DE VAISSEAUX SANGUINS

(30) Priority: 31.03.2017 RU 2017104521
(43) Date of publication of application: 05.02.2020
(73) Proprietor: AO Medtekhnoproekt, Moscow 143026 (RU)
(72) Inventor: SEDELNIKOV, Nikolai Georgievich, Moscow 115561 (RU); BEKBAEV, Almaz Serikovich, Moscow 117342 (RU); ROMANOVA, Irina Viktorovna, Pushkin 196602 (RU)
(74) Representative: Berggren Oy
(86) International application number: PCT/RU2018/050034
(87) International publication number: WO 2018/182462

(56) References cited:
- WO-A1-01/58504
- US-A- 5 207 706
- PISKAREV M.S. et al.: "Surface modification of films of fluorine-containing polymers in low-temperature plasma", V International Symposium on Theoretical and Applied Plasma Chemistry. Collection of works; September 3-8, 2008; Ivanovo, RU, vol. 2, 2008, pages 384-387, XP009517000,

## Description

### Technical field

This invention relates to medicine and medical equipment, in particular to a technology for coating medical implantable devices placed inside the patient's body and directly to the said devices having at least one surface in contact with blood, in particular, to blood vessel prostheses made of a polymeric material (polyethylene terephthalate). Using the invention allows to activate the endothelization process and prevent thrombosis.

**Background of the Invention**Death caused by cardiovascular diseases is leading the world in the causes of death. As vascular surgery develops, more and more patients with cardiovascular diseases undergo reparative and reconstructive surgery. The treatment of cardiac pathologies is often performed using autogenetic grafts, however, in many cases, the use of synthetic prostheses of various designs is preferable. In modern reparative and reconstructive surgery of the heart and blood vessels, implants made of polymeric materials are widely used, which must possess the required morphological, physical and mechanical and surface properties.

Within the past 15-20 years, vascular prostheses based on polyethylene terephthalate (PET, Dacron, RET) have gained the greatest popularity in clinical practice. This is the most chemically stable and biologically inert polymer material, therefore implantation of prostheses made from it causes a minimal reaction of surrounding tissues. The greater stiffness of the dacron threads is an advantage in creating stable mechanical structures. At the same time, the technology used for manufacturing of knitted prostheses makes it possible to obtain tubes whose plasticity exceeds the plasticity of PTFE prostheses. The gap between the threads (fibers) of the prosthesis enables germination of endothelial cells through the knitted structure of the prosthesis. In this case, the material basically does not change its biomedical and physicochemical properties for a long time under the influence of biological media *in vivo.* However, when using prostheses made of polymeric materials, in particular PET, there are problems related to increased thrombosis.

From the onset of using the polymer medical devices, efforts have been made to increase their biocompatibility and, in particular, to create an antithrombogenic, fibrinolytic or thrombolytic interface with such body fluids, such as blood. For this purpose, various methods for increasing the thromboresistance of polymer products are being developed, namely, coatings for polymer prostheses are developed.

In the prior art, the method for endothelization of blood vessels prostheses *in vitro* is known, which is described in RU2205612. The above method includes electrification of the prosthesis inner surface, thermal stabilization of charges at a temperature of 150°C, filling the prosthesis with a suspension of endothelium in a nutrient medium, and fixing endothelial cells on the inner surface of the prosthesis. The patient's autogenous endothelium or human endothelium at the stage of intrauterine development is used as the endothelium. However, the disadvantage of this method is that the coating obtained by this method is nondurable and is easily washed off with blood. The complexity and duration of the prosthesis modernization process using the proposed method makes its serial implementation significantly laborious.

US 5744515 contains information about an implantable medical device made of rigid porous biomaterial, on the surface of which molecules of fibronectin, laminin, and collagen are immobilized, which contribute to *in vivo* capillary endothelization of the device.

From document US6322588, a medical device is known that is in contact with liquids in a patient's body, including blood, and which is a polymer substrate coated with a metal. Inert metals such as titanium, cobalt, nickel and others are used as coatings. However, due to the fact that the coating of such prostheses is large and solid, it prone to cracking and crumbling during prolonged use in the blood stream and does not contribute to the growth of the endothelium on the surface of the prosthesis.

From document US20030050691, an implantable device is known, as well as its construction method. The implantable device is a vascular prosthesis, characterized in that it is made of a polymer substrate with a metal coating. The preferred coating material is a solid titanium layer with a thickness of 50-300 nm with a substantially X-ray amorphous structure. The disclosure provides only short-term thrombotic resistance of the prosthesis, since it does not lead to endothelization and the formation of neointima on the entire surface of the artificial vessel contacting with blood. WO0158504 discloses a method for applying a non-continuous coating on cardiovascular devices comprising applying a metal coating by magnetron sputtering.

Thus, a need to develop vascular prostheses having a stable antithrombogenic coating and a method for applying such a coating to polymeric vascular prostheses made of polyethyleneterephthalate still remains. The development of methods for the manufacture and modification of polymers and products based on them will help to make transition to solving the most important problems of theoretical and practical cardiology.

### Disclosure of the invention

The goal of the invention is to develop a method for creating a discontinuous coating for polymeric vascular prostheses made of polyethylene terephthalate, which allows to obtain a bio- and hemo-compatible coating of prostheses characterized by low thrombogenicity, as well as creating a vascular prosthesis with a stable antithrombogenic coating.

The technical result of this invention is to reduce the time of neointima formation over the entire inner surface of the vascular prosthesis due to the technology of applying a discontinuous coating on the prosthesis inner or outer surface. In addition, the coating applied to the prosthesis by the method proposed by the invention is characterized by high stability, does not laminate from the prosthesis and is not subject to cracking, due to which vascular prostheses having such a coating are characterized by durable antithrombogenicity.

The achievement of the specified technical result is ensured by the implementation of the method of applying a discontinuous coating of metal of the IVB or VB group on the inner and outer surfaces of the polymeric prosthesis of a blood vessel made of polyethylene terephthalate, which includes the following steps:
- plasma-chemical modification of the polymer prosthesis surfaces;
- coating by magnetron sputtering method with an unbalanced plasma in an argon-oxygen plasma;
in that, the coating is carried out cyclically by alternating of ion etching and magnetron sputtering coating.

In particular embodiments options of the invention, a polyethylene terephthalate blood vessel prosthesis has at least one surface that is in contact with blood.

In particular embodiments options of the invention, one cycle includes coating by magnetron sputter with an unbalanced plasma for 30 seconds and subsequent ion etching for 10 seconds.

In particular embodiments of the invention, the coating is applied within four cycles.

In particular embodiments of the invention, the coating by the magnetron sputtering method with an unbalanced plasma in an argon-oxygen plasma is carried out at a pressure of 2±0.5 Pa and an Ar:O₂ = 1: 9 ratio, with a pellet sputter power density of 5-6 W/cm².

In particular embodiments options of the invention, the method includes a preliminary stage of cleaning the polymer prosthesis in an ultrasonic bath with a bactericidal substance and/or a stage of ion cleaning (ion etching) in an argon atmosphere. In some preferred options, the bactericidal substance is benzalkonium chloride or chlorhexidine.

In particular embodiments options of the invention, the stage of preliminary ion purification (ion etching) in an argon atmosphere, is carried out for 50-60 seconds.

In particular embodiments options of the invention, the plasma-chemical modification of the polymer prosthesis is carried out using ammonia in a high-frequency 13.56 MHz discharge with a power of 20-40 W, and a pressure of 6.6 Pa for 45-60 minutes.

In particular embodiments options of the invention, ion etching is carried out using a slotted ion source with uneven ion current density along the length of the treated object ± 10% under the following modes:
- operating voltage - 2 kV;
- operating discharge current in argon - 0.5 A.

In particular embodiments options of the invention, the coating metal is either titanium, or tantalum, or zirconium, or niobium.

In particular embodiments of the invention, the coating particle size is 20-100 nm.

The technical result is also achieved by creating a prosthesis of a blood vessel containing a frame made of polyethylene terephthalate, with internal and external surfaces, on the internal and external surfaces of which a discontinuous coating of a metal of IVB or VB group is applied by the above method.

In particular embodiments options of the invention, the prosthesis coating metal is either titanium, or tantalum, or zirconium, or niobium.

In particular embodiments of the invention, the coating particle size is 20-100 nm.

### Terms and Definitions

In this document, the term **"surface cleanliness"** means as a slight surface roughness.

In this document, the term **"surface roughness"** means a combination of surface irregularities with relatively small steps at a base length that determines the operational characteristics of the surface. It must be understood that at any method of manufacturing the parts surfaces cannot be absolutely smooth, because traces of processing are left on them, which are basically alternating protrusions and depressions of various geometric shapes and sizes.

In this document, the term **"prosthesis"** means, in particular, a vascular prosthesis having a frame made of PET fibers (Dacron^{®}), usually woven, knitted or braided, and the fibers are, consequently, arranged in a certain geometry and structure, which renders it suitable mechanical properties, including porosity. The geometric shape of the frame is preferably made cylindrical.

The term **"discontinuous coating"** in this document should mean a coating formed from particles of a size from several dozens to 200 nm, in particular versions up to 100 to nm, which, as a rule, have a shape close to spherical, more precisely, capable of fitting into the sphere, and do not fuse with each other. The crystal seeds in the initial period of this coating formation on the surface of a foreign substrate, grow to a certain size, while there remains a gap between the seeds, although there may be points of contact. Further growth leads to their fusion and the formation of a continuous film (coating), which should not be allowed, according to the invention, since continuous coatings inevitably cause stresses that can lead to cracking or flaking. In the case of non-fused coating particles, this is excluded.

### Brief Description of the Drawings

**Figure 1****.** Polyethylene terephthalate (PET) fibers without coating.
**Figure** 2. A titanium coating deposited on a knitted Dacron substrate by a method without alternating (interrupting) the process of sputtering by ion etching, the sputtering time is 30 seconds (lamination of the coating).
**Figure 3****.** A titanium coating deposited on a knitted Dacron substrate by a method without alternating (interrupting) the ion etching deposition process, the deposition time is 70 seconds (coating cracking).
**Figure 4****.** ePTFE Surface (Polytetrafluoroethylene) prosthesis without coating.
**Figure 5****.** Titanium coating of a PET prosthesis obtained by the invention method.

### Detailed Disclosure of Invention

To increase the service period of the surgically reconstructed vessels, it is required to minimize the risk of prostheses thrombosing. The unimpaired operation of the vascular prosthesis is possible only after the appearance of the endothelial lining, which synthesizes anticoagulant factors and prevents the growth of smooth muscles, and, consequently, prevents a decrease in the lumen of the vessel. The accelerated buildup of neointima on the inner surface of the prosthesis of a blood vessel is especially important under conditions when the potentials for regeneration are already reduced.

Endothelization can be carried out by two different mechanisms. According to one mechanism called transanastamotic (from anastomosis), endothelization occurs from the internal cavity of the blood vessel into which the prosthesis is placed. As a result, endothelial cells line the lumen of the prosthesis, migrating from the line of the anastomosis. Another mechanism is transmural, i.e. penetrating the wall or intermediate tissue, which activates the endothelial cells growth through the walls of the prosthesis.

The coating applied on the prosthesis of polyethylene terephthalate (PET) in accordance with the invention, ensures accelerated formation of neointima, since the endothelization process is carried out by the two above-mentioned mechanisms simultaneously. The method of the invention differs in that the coating is applied both on the external and on the internal surfaces of the prosthesis. Coating on the internal surface of the prosthesis allows the endothelial cells to line the lumen of the prosthesis through migration from the anastomotic line. The coating on the external surface of the prosthesis is applied in order to accelerate the formation of the endothelial layer inside the prosthesis due to the germination of endothelial cells through the porous knitted base of the prosthesis and the formation of a lining in the area remote from the anastomosis. Thus, when implanting the prosthesis, the endothelium spreads simultaneously from both the anastomosis and transmurally from the external surface of the prosthesis, through the pores.

As noted it was above, to activate the process of endothelial cells migration from the anastomosis and from the surface of the prosthesis that is not in contact with blood, their fixing and increase the adhesive strength of the growing endothelial layer in accordance with the invention, a discontinuous coating based on metals of IVB or VB groups, for example, based on Ti, Zr, Nb or Ta is sequentially applied both from the inside and from the outside on the prosthesis material (first from the inside, and then from the outside)

The discontinuous coating applied by the invention method, activating the endothelization process, consists of particles which are several tens of nanometers in size. A further increase in the particle size of the coating leads to the formation of a continuous coating, which is an obstacle to the relaxation of emerging stresses and increases the likelihood of the coating lamination.

A coating with a set particle size is applied by magnetron sputtering with an unbalanced plasma, which contributes to the maximum penetration of the sprayed particles into the through pores and the surface irregularities of the prosthesis material, cyclically in the mode of alternation with ion etching.

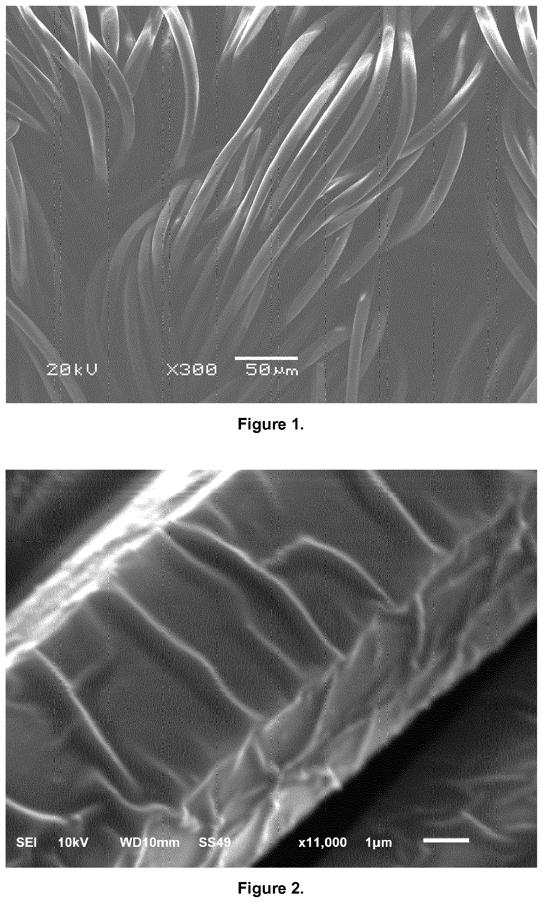

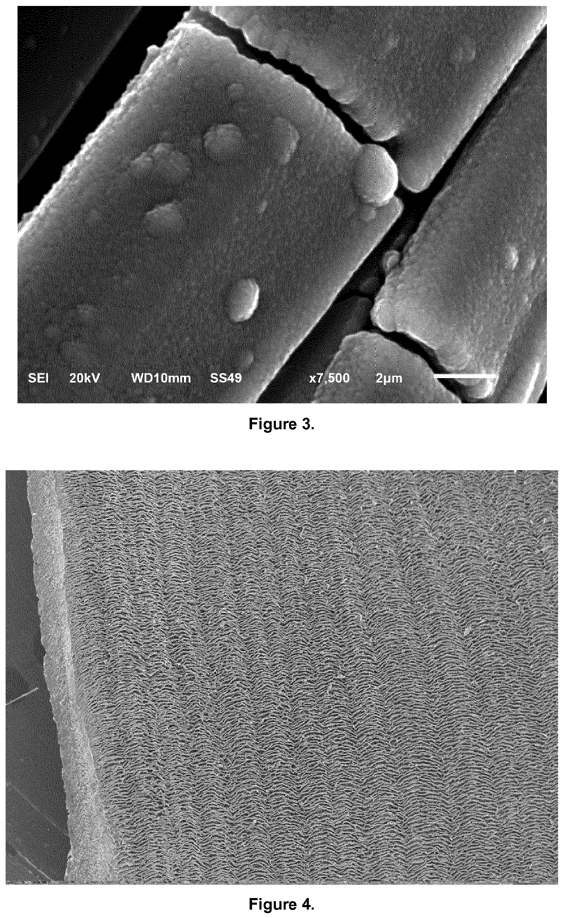

The main condition for the successful functioning of an artificial vessel is the rapid growth and fixing of endothelial cells on its internal surface. This is possible due to thermodynamic instability of the conglutination surface, i.e. increased surface Gibbs energy. The energy of the surface layer, or surface energy Gs, is proportional to the interfacial area (s): Gs = σ·s, where the proportionality coefficient σ is the surface strain. To fulfill this condition, the internal surface of the prosthesis must be developed, since obtaining a developed or more developed surface means an increase in roughness, i.e. increase in interfacial area. At the same time, the coating applied on the vessel surface must be discontinuous so as not to create large stresses that can lead to lamination and cracking (fig. 2-3). Since PET fibers (fig. 1) are characterized by high surface cleanliness (fig. 4 shows the image of the prosthesis ePTFE surface for comparison), i.e., the metal deposited from the gas phase forms a continuous layer at an arbitrarily small controlled coating thickness due to a low roughness and a vanishingly low concentration of crystallization centers, which, as previously was noted, leads to coating lamination and cracking. Unexpectedly, modes of alternating ion etching with magnetron sputtering were found experimentally to limit the formation of crystallization centers by removing small crystalline nuclei by ion etching.

As a rule, polymeric materials are characterized by low surface energy values, are poorly wetted by solvents, have low adhesion to sprayed metal layers, etc. In this invention, in order to overcome these limitations, the surface of the polymer prosthesis undergoes a plasmochemical modification involving a very thin layer not exceeding 100 Å, while the bulk of the polymer does not change, while maintaining the mechanical, physicochemical and electrophysical properties of the material modified. The effect of plasma on the polymer surface allows to change mainly its contact properties (wetting, adhesion) by way of hydrophilic groups formation. At the same time, surface cleaning of the adsorbed molecular layer is achieved. The process is carried out using ammonia or its mixtures with hydrogen, as a result of which nitrogen-containing groups appear on the surface (amino, amido, imido, imino, etc.).

### Implementation of the Invention

The ability to objectively achieve a technical result in the invention implementation is confirmed by reliable data given in the examples containing experimental data. It should be understood that these and all examples cited in the application materials are not limiting and are provided merely to illustrate the present invention.

The coating of the method of the invention, which is defined by the claims, is obtained by magnetron sputtering with an unbalanced pellet plasma (a metal plate, for example, made of titanium, tantalum, zirconium or niobium), for example, of titanium grade VT-1-0 (or ASTM F67). The composition and structure of the coating deposited on substrates (for example, polyethylene terephthalate in the form of plates or DALLON^{®}H tubes manufactured by TRICOMED) depends on the gas atmosphere in the sputter chamber and the process conditions.

Before the start of the sputtering process, the substrate is subject to cleaning in an ultrasonic bath with highly effective bactericidal substances characterized by a wide spectrum of activity and low toxicity, such as benzalkonium chloride or chlorhexidine. The dried substrate (polymer prosthesis) is placed in a vacuum chamber, which is pumped out to P = 0.1 Pa.

This is followed by ion purification in an argon atmosphere (Ar) at a residual pressure of 0.1-1.0 Pa. The purpose of ion cleaning is to create a juvenile (ideally clean and active, i.e. with unblocked crystallization centers) surface of the substrate. Therefore, it is necessary to apply coating in vacuum and after ionic cleaning of the base.

For this purpose, a slot ion source with an inhomogeneity of ion current density of ± 10% is mounted in a vacuum chamber. The process is carried out at a current of argon ions of 0.5 A and a voltage of 2 kV.

Further, the surface of the polymer prosthesis undergoes a plasma-chemical modification. The process is carried out using ammonia in a high-frequency 13.56 MHz discharge with a power of 20-40 W, a pressure of 6.6 Pa for 45-60 minutes.

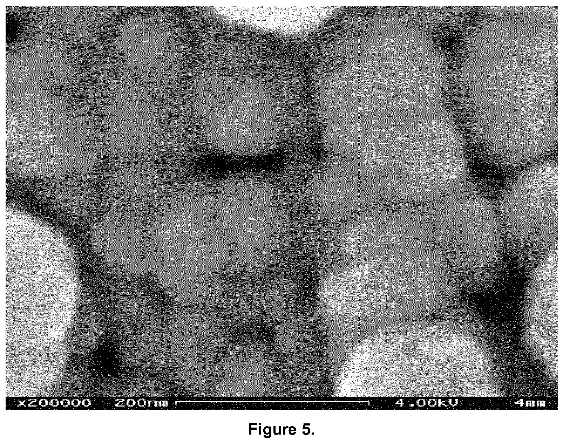

The coating, according to the invention process, is applied by magnetron sputtering in argon-oxygen plasma, in particular, at a pressure of 2 ± 0.5 Pa and an ratio Ar:O2 = 9:1 (the mode is optimal for obtaining the most developed surface). The deposition of the coating in argon leads to the formation of a structure characterized by increased porosity. The nitrogen content in the coating should be minimized, since its presence leads to reduction (reduction of the coating particle size) and compaction of the structure. The size of the particles forming the coating, as a rule, does not exceed 20-100 nm (fig. 5). Plasma-forming gas was supplied directly to the discharge zone in order to reduce surface contamination. The unbalanced magnetron spray system is used (for example, the unbalanced magnetron spray system NM-V-65MK, manufactured by NPF Elan-Practik). The planar magnetron device was supplied from an independent constant voltage source. The sputtering magnetron provides sputtering of the pellet metal over the surface of the substrate with uniformity of at least ± 10%.

The optimal value of the pellet sputter power density is in the range of 5-6 W/cm². The spraying rate is 3-5·10⁻⁴ g/min.cm². Spraying is carried out in 4 cycles, each of which includes sputtering of the coating for 30 seconds and subsequent ion etching for 10 sec. when the value of current of argon ions equal to 0.5 A.

In this case, visual control of the discontinuity of the applied coating is carried out. The theoretical maximum allowable specific weight gain of the titanium coating - 5·10⁻³ mg/cm².

The application of discontinuous coating, in accordance with the invention process, was carried out on the surface of the vascular polymer prosthesis stagewise: first, the coating was applied to the internal surface in 4 cycles, and then - to the external surface in 4 cycles.

The specified discontinuous coating applied on the prosthesis made of polyethylene terephthalate by the invention method, is characterized by high stability, does not laminate from the prosthesis and is not subject to cracking.

Unlike a continuous coating, which tends to laminate from the prosthesis in the entire zone of contact with the vessel, the destruction of the discontinuous coating is localized in the area of a single section, which leads to discontinuous coating non-lamination from the prosthesis over its entire surface.

### In vitro assessment of cell adhesion to the surface of prosthetic material.

The study of cell adhesion to the surface of the samples and their survival, as well as an assessment of the proliferation rate of cell culture, were performed using an *in vitro* test system. *In vitro* assessment of cell activity is performed in cultural plates on samples of strictly defined shape and size. To assess the proliferation of endotheliocytes, a standard MTT test was used. Four types of samples based on polyethylene terephthalate were investigated:
1) Dacron (Dacron knitted DALLON^{®}H) without coating;
2) Dacron with a one-sided titanium coating, and the cells were populated on the surface with a titanium coating (*);
3) Dacron with a one-sided titanium coating, and the cells being populated by the surface without a titanium coating (*);
4) Dacron with biopolymer (based on collagen IV) coating. (*) Cells of the line EA.hy926 were populated on a surface containing a titanium coating in the vicinity of pores in a knitted substrate.

Cells of the EA.hy926 line were used as a model cell culture for assessing the matrix properties of the samples under study. This is one of the most commonly used lines obtained by fusion of HUVEC and human lung adenocarcinoma A549 cell line. Cells were cultured in growth medium (DMEM / F-12 (Dulbecco's Modified Eagle Medium / Nutrient Mixture F-12) (1: 1) supplemented with 10% fetal bovine serum, 100 units/ml penicillin, 100 µg/ml streptomycin (all the reagents listed are Gibco)) in a CO₂ incubator under standard conditions (37°C, 5% CO₂, 80% humidity). Cells were removed from the plastic surface by incubation in 0.25% trypsin solution mixed with Versen solution (1:1) at 37°C for 5 min, after which the cells were precipitated by centrifugation and resuspended in growth medium.

24 hours before the colonization of the samples with cells, the wells of the plates were filled with growth medium to wet the samples; immediately before the introduction of cells, the medium was taken from the wells. Cells were transferred into a suspension, as described above, and then applied on samples (4 types specified above) aliquots of the cell suspension in 1 ml of growth medium (cell concentration 200,000 cells/ml). Further cultivation was carried out in a CO₂ incubator under standard conditions for a maximum of five days. In 6, 24, 72, and 120 hours after colonization, the number of viable cells attached to the samples was estimated. For this purpose, we used the MTT test method, which allows a comprehensive assessment of proliferative activity, consisting of such indicators as the ratio of living and dead cells, cell population growth dynamics, and the monolayer formation rate. Upon reaching the experiment control time, 100 µl of 3- (4,5-dimethylthiazol-2-yl) -2,5-diphenyl tetrazolium bromide solution at a concentration of 5 mg/ml was added to the wells of the plate (final concentration was 0.5 mg/ml), after which they were incubated for another 3 hours. At the end of the procedure, the samples were removed, washed in phosphate-buffered saline, and then placed on a plate, the wells of which contained 200 µl of dimethyl sulfoxide (DMSO) and shaken on an automatic shaker for 5 min. for complete dissolution of formazan crystals. The optical density of the wells contents was measured on an Infinite 200 PRO flatbed scanner (Tecan, Germany) at a wavelength of 550 nm, a reference measurement was carried out at 690 nm.

**Table 1. The concentration (× 10³ml⁻¹) of the cell culture subject to the experiment duration (the table shows the average values obtained from 5 measurements).**

| **Substrate type** | **Time, h** | | | |
|---|---|---|---|---|
| | **6** | **24** | **72** | **120** |
| **1** | 43.21±3.04 | 48.47±3.90 | 77.44±7.06 | 103.71±9.97 |
| **2** | 42.36±3.51 | 72.84±7.49 | 160.64±13.06 | 319.22±16.78 |
| **3** | 43.74±1.34 | 52.50±3.22 | 112.52±8.16 | 151.30±7.21 |
| **4** | 44.08±1.45 | 51.4±1.34 | 119.24±10.45 | 101.7±11.54 |

As can be seen from table 1, after 6 hours of colonization, the number of viable cells on samples of different types was leveled. The difference in the adhesion rate of EA.hy926 cells on all samples is not statistically significant (43.21±3.04, 42.36±3.51, 43.74±1.34, 44.08±1.45). However, measurements after 3 and 5 days indicated a significant excess of the proliferation rate of EA.hy926 cells for samples with a titanium coating: within a period of 6-120 hours, it amounted to 7.54±0.75. For samples with a titanium layer in the region of pores in knitted dacron fabric, this indicator is equal to 3.46±0.16, which also exceeds the proliferation rate of samples No. 4 and No.1: 2.31±0.09 and 2.40±0.17, respectively.

Based on the presented research results, we can infer the following:
1) A sample made from Dacron (PET) ensures adhesion of EA.hy926 cell lines to its surface and allows them to maintain viability, but does not contribute to cell proliferative activity at their further cultivation;
2) Modification of a sample made from Dacron (PET) by sputtering titanium by the invention method increases the number of endothelial cells attached to the surface of the samples and maintains a stable high rate of endothelial cells proliferation;
3) The transmural mechanism of endothelization makes a significant contribution to the neointima formation on the internal surface of the prosthesis made of Dacron (PET), distant from the anastomosis line, which can lead to a significant reduction in the time of neointima formation over the entire internal surface of the prosthesis and prevention of thrombosis.

Although the invention has been described with reference to the disclosed invention embodiment options, it should be apparent to specialists of this area that the specific experiments described in detail are provided for illustrative purposes only and should not be construed as limiting the scope of the invention, which is defined by the claims, in any way.

## Claims

1. A method of applying a discontinuous coating of a metal of IVB or VB group on the internal and external surfaces of a polymeric prosthesis of a blood vessel made of polyethylene terephthalate, including the following steps:
- plasma-chemical modification of the polymeric prosthesis;
- coating by magnetron sputtering method with an unbalanced plasma in an argon-oxygen plasma; wherein the coating is applied cyclically in the mode of alternating the process of magnetron sputtering with the process of ion etching.

2. The method according to claim 1, wherein the polyethylene terephthalate blood vessel prosthesis has at least one surface contacting with blood.

3. The method according to claim 1, wherein the coating is applied in 4 cycles.

4. The method according to claim 1, wherein each cycle includes magnetron sputtering of a coating with an unbalanced plasma in an argon-oxygen plasma for 30 seconds and subsequent ion etching for 10 seconds.

5. The method according to claim 1, wherein the application of a discontinuous coating by magnetron sputtering with an unbalanced plasma in an argon-oxygen plasma is performed at a pressure of 2 ± 0.5 .Pa and an Ar: O₂ ratio of 9:1, with a pellet sputter power density of 5-6 W/cm²

6. The method according to claim 1, wherein the plasma-chemical modification of the polymer prosthesis is performed using ammonia in a high-frequency 13.56 MHz discharge with a power of 20-40 W, a pressure of 6.6 Pa for 45-60 minutes.

7. The method according to claim 1, which additionally includes pre-cleaning the surface of the polymer prosthesis in an ultrasonic bath and/or ion cleaning in an argon atmosphere.

8. The method according to claim 7, wherein ion cleaning is performed within 50-60 seconds.

9. The method according to claim 1, wherein ion etching is performed using a slot ion source with uneven ion current density along the length of the treated object ± 10% under the following conditions:
- operating voltage - 2 kV;
- operating discharge current in argon - 0.5 A.

10. The method according to claim 1, wherein the metal is either titanium, or tantalum, or zirconium, or niobium.

11. The method according to claim 1, wherein the particle size of the coating is 20-100 nm.

12. A blood vessel prosthesis comprising a polyethylene terephthalate frame with internal and external surfaces, wherein a non-continuous IVB or VB group metal coating is applied on the internal and external surfaces of the frame using the method of claim 1.

13. The prosthesis according to claim 12, wherein the metal is either titanium, or tantalum, or zirconium, or niobium.

14. The prosthesis according to claim 12, wherein the particle size of the coating metal is 20-100 nm.

## Patentansprüche

1. Verfahren zum Aufbringen einer diskontinuierlichen Beschichtung aus einem Metall der Gruppe IVB oder VB auf die innen- und außenliegenden Flächen einer polymerbasierten Prothese eines Blutgefäßes, die aus Polyethylenterephthalat hergestellt ist, wobei es die folgenden Schritte beinhaltet:
- plasmachemische Modifizierung der polymerbasierten Prothese;
- Beschichten mittels des Magnetron-Sputterverfahrens mit einem Nichtgleichgewichtsplasma in einem Argon-Sauerstoff-Plasma;
wobei das Beschichten auf zyklische Weise derart zur Anwendung gebracht wird, dass der Vorgang des Magnetronsputtern im Wechsel mit dem Vorgang des Ionenätzens erfolgt.

2. Verfahren gemäß Anspruch 1, wobei die Blutgefäßprothese aus Polyethylenterephthalat mindestens eine Fläche aufweist, die mit Blut in Kontakt kommt.

3. Verfahren gemäß Anspruch 1, wobei die Beschichtung in 4 Zyklen aufgebracht wird.

4. Verfahren gemäß Anspruch 1, wobei jeder Zyklus ein Magnetronsputtern einer Beschichtung mit einem Nichtgleichgewichtsplasma in einem Argon-Sauerstoff-Plasma über einen Zeitraum von 30 Sekunden und anschließend ein lonenätzen über einen Zeitraum von 10 Sekunden umfasst.

5. Verfahren gemäß Anspruch 1, wobei das Aufbringen einer diskontinuierlichen Beschichtung mittels Magnetron-Sputtern mit einem Nichtgleichgewichtsplasma in einem Argon-Sauerstoff-Plasma bei einem Druck von 2 ± 0,5 Pa und einem Ar/O₂-Verhältnis von 9:1 durchgeführt wird, mit einer pelletbezogenen Sputter-Leistungsdichte von 5 bis 6 W/cm².

6. Verfahren gemäß Anspruch 1, wobei die plasmachemische Modifizierung der Polymerprothese unter Verwendung von Ammoniak in einer Hochfrequenzentladung von 13,56 MHz mit einer Leistung von 20 bis 40 W, einem Druck von 6,6 Pa über einen Zeitraum von 45 bis 60 Minten durchgeführt wird.

7. Verfahren gemäß Anspruch 1, wobei es zusätzlich dazu ein Vorreinigen der Fläche der Polymerprothese in einem Ultraschallbad und/oder ein lonenstrahlreinigen in einer Argonatmosphäre umfasst.

8. Verfahren gemäß Anspruch 7, wobei das lonenstrahlreinigen innerhalb von 50 bis 60 Sekunden durchgeführt wird.

9. Verfahren gemäß Anspruch 1, wobei das lonenätzen unter Verwendung einer schlitzartigen lonenquelle mit einer lonenstromdichte, die über die Länge des behandelten Gegenstand eine Ungleichmäßigkeit von ± 10% aufweist, unter den folgenden Bedingungen durchgeführt wird:
- Betriebsspannung - 2 kV;
- Betriebsentladungsstrom in Argon - 0,5 A.

10. Verfahren gemäß Anspruch 1, wobei es sich bei dem Metall entweder um Titan oder um Tantal oder um Zirconium oder um Niob handelt.

11. Verfahren gemäß Anspruch 1, wobei die Partikelgröße der Beschichtung 20 bis 100 nm beträgt.

12. Blutgefäß-Prothese, die einen Polyethylenterephthalat-Rahmen mit innen- und außenliegenden Flächen umfasst, wobei eine nichtkontinuierliche Beschichtung aus einem Metall der Gruppe IVB oder VB unter Anwendung des Verfahrens nach Anspruch 1 auf die innen- und außenliegenden Flächen des Rahmens aufgebracht wird.

13. Prothese gemäß Anspruch 12, wobei es sich bei dem Metall entweder um Titan oder um Tantal oder um Zirconium oder um Niob handelt.

14. Prothese gemäß Anspruch 12, wobei die Partikelgröße des Beschichtungsmetalls 20 bis 100 nm beträgt.

## Revendications

1. Procédé destiné à l'application d'un revêtement discontinu d'un métal du groupe IVB ou VB sur les surfaces interne et externe d'une prothèse polymère d'un vaisseau sanguin, réalisée à partir de polyéthylène téréphtalate, qui comprend les étapes suivantes dans lesquelles :
- on soumet la prothèse polymère à une modification plasmachimique ;
- on procède à une enduction par l'intermédiaire d'un procédé de pulvérisation cathodique magnétron avec un plasma dissymétrique dans un plasma d'argon-oxygène ;
dans lequel le revêtement est appliqué de manière cyclique dans le mode d'alternance du processus de pulvérisation cathodique magnétron avec le processus de gravure ionique.

2. Procédé selon la revendication 1, dans lequel la prothèse du vaisseau sanguin en polyéthylène téréphtalate possède au moins une surface qui entre en contact avec le sang.

3. Procédé selon la revendication 1, dans lequel le revêtement est appliqué au cours de 4 cycles.

4. Procédé selon la revendication 1, dans lequel chaque cycle englobe une pulvérisation cathodique magnétron d'un revêtement avec un plasma dissymétrique dans un plasma d'argon-oxygène pendant 30 secondes et une gravure ionique ultérieure pendant 10 secondes.

5. Procédé selon la revendication 1, dans lequel l'application d'un revêtement discontinu par l'intermédiaire d'une pulvérisation cathodique magnétron avec un plasma dissymétrique dans un plasma d'argon-oxygène est mise en œuvre sous une pression de 2 ± 0,5 Pa. et un rapport Ar : O₂ qui s'élève à 9 : 1, avec une densité de puissance surfacique lors de la pulvérisation des pastilles qui s'élève à 5 - 6 W/cm².

6. Procédé selon la revendication 1, dans lequel la modification plasmachimique de la prothèse polymère est mise en œuvre en utilisant de l'ammoniac dans une décharge à haute fréquence de 13,56 MHz avec une puissance de 20 à 40 W, une pression de 6,6 Pa pendant un laps de temps de 45 à 60 minutes.

7. Procédé selon la revendication 1, qui comprend en outre un nettoyage préalable de la surface de la prothèse polymère dans un bac à ultrasons et/ou un nettoyage par ions dans une atmosphère à base d'argon.

8. Procédé selon la revendication 7, dans lequel le nettoyage par ions est mis en œuvre dans un laps de temps de 50 à 60 secondes.

9. Procédé selon la revendication 1, dans lequel la gravure ionique est mise en œuvre en utilisant une source d'ions sous la forme d'une fente avec une densité du courant ionique inégale sur la longueur de l'objet traité à concurrence de ± 10 % dans les conditions indiquées ci-après :
- une tension de service de ∼ 2 kV ;
- un courant de décharge de service dans de l'argon de ∼ 0,5 A.

10. Procédé selon la revendication 1, dans lequel le métal représente soit du titane, soit du tantale, soit de zirconium, soit du niobium.

11. Procédé selon la revendication 1, dans lequel la granulométrie du revêtement s'élève de 20 à 100 nm.

12. Prothèse destinée à un vaisseau sanguin qui comprend une structure en polyéthylène téréphtalate munie de surfaces interne et externe ; dans laquelle on applique un revêtement non continu d'un métal du groupe IVB ou VB sur les surfaces interne et externe de la structure en utilisant le procédé selon la revendication 1.

13. Prothèse selon la revendication 12, dans laquelle le métal représente soit du titane, soit du tantale, soit de zirconium, soit du niobium.

14. Prothèse selon la revendication 12, dans laquelle la granulométrie du métal du revêtement s'élève de 20 à 100 nm.
